# FASCICULE DE BREVET EUROPEEN

(11) **EP 2 274 623 B1**
(45) Date de publication et mention de la délivrance du brevet: **12.10.2011**
(21) Numéro de dépôt: 09742310.7
(22) Date de dépôt: 10.04.2009
(51) Int. Cl.: G01N 33/68

(54) **PROCEDE DE DIAGNOSTIC D'UNE HYPERTENSION ARTERIELLE PULMONAIRE**
DIAGNOSEVERFAHREN FÜR PULMONALE ARTERIELLE HYPERTONIE
METHOD FOR DIAGNOSING PULMONARY ARTERY HYPERTENSION

(30) Priorité: 11.04.2008 FR 0852459
(43) Date de publication de la demande: 19.01.2011
(73) Titulaire: Assistance Publique Hôpitaux De Paris, 75001 Paris (FR); Université Paris Descartes, 75270 Paris Cedex 06 (FR); UNIVERSITE PARIS-SUD 11, 91405 Orsay Cedex (FR)
(72) Inventeur: MOUTHON, Luc, F-94160 Saint Mandé (FR); HUMBERT, Marc, F-92130 Issy les Moulineaux (FR); TAMBY, Mathieu, F-93600 Aulnay sous Bois (FR)
(74) Mandataire: Chajmowicz, Marion
(86) Numéro de dépôt international: PCT/FR2009/050661
(87) Numéro de publication internationale: WO 2009/136112

(56) Documents cités:
- WO-A-99/43308
- WO-A-2005/009366
- JEFFERY ET AL: "Molecular and cellular basis of pulmonary vascular remodeling in pulmonary hypertension" PROGRESS IN CARDIOVASCULAR DISEASES, SAUNDERS, PHILADELPHIA, PA, US, vol. 45, no. 3, 1 novembre 2002 (2002-11-01), pages 173-202, XP005128803 ISSN: 0033-0620
- IHIDA-STANSBURY KAORI ET AL: "Tenascin-C is induced by mutated BMP type II receptors in familial forms of pulmonary arterial hypertension" AMERICAN JOURNAL OF PHYSIOLOGY - LUNG CELLULAR AND MOLECULAR PHYSIOLOGY, vol. 291, no. 4, octobre 2006 (2006-10), pages L694-L702, XP002502458 ISSN: 1040-0605

### SEQUENCE LISTING

<110> Assistance publique Hopitaux de Paris
<120> Procédé de diagnostic d'une hypertension artérielle pulmonaire
<130> B0700
<160> 1
<170> PatentIn version 3.3
<210> 1
   <211> 2201
   <212> PRT
   <213> Homo sapiens
<220>
   <221> MISC_FEATURE
   <222> (181) .. (290)
   <223> fragment antigénique
<400> 1
   Met Gly Ala Met Thr Gln Leu Leu Ala Gly Val Phe Leu Ala Phe Leu
   1               5                   10                  15
   Ala Leu Ala Thr Glu Gly Gly Val Leu Lys Lys Val Ile Arg His Lys
               20                  25                  30
   Arg Gln Ser Gly Val Asn Ala Thr Leu Pro Glu Glu Asn Gln Pro Val
           35                  40                  45
   Val Phe Asn His Val Tyr Asn Ile Lys Leu Pro Val Gly Ser Gln Cys
       50                  55                  60
   Ser Val Asp Leu Glu Ser Ala Ser Gly Glu Lys Asp Leu Ala Pro Pro
   65                  70                  75                  80
   Ser Glu Pro Ser Glu Ser Phe Gln Glu His Thr Val Asp Gly Glu Asn
                   85                  90                  95
   Gln Ile Val Phe Thr His Arg Ile Asn Ile Pro Arg Arg Ala Cys Gly
               100                 105                 110
   Cys Ala Ala Ala Pro Asp Val Lys Glu Leu Leu Ser Arg Leu Glu Glu
           115                 120                 125
   Leu Glu Asn Leu Val Ser Ser Leu Arg Glu Gln Cys Thr Ala Gly Ala
       130                 135                 140
   Gly Cys Cys Leu Gln Pro Ala Thr Gly Arg Leu Asp Thr Arg Pro Phe
   145                 150                 155                 160
   Cys Ser Gly Arg Gly Asn Phe Ser Thr Glu Gly Cys Gly Cys Val Cys
                   165                 170                 175
   Glu Pro Gly Trp Lys Gly Pro Asn Cys Ser Glu Pro Glu Cys Pro Gly
               180                 185                 190
   Asn Cys His Leu Arg Gly Arg Cys Ile Asp Gly Gln Cys Ile Cys Asp
           195                 200                 205
   Asp Gly Phe Thr Gly Glu Asp Cys Ser Gln Leu Ala Cys Pro Ser Asp
       210                 215                 220
   Cys Asn Asp Gln Gly Lys Cys Val Asn Gly Val Cys Ile Cys Phe Glu
   225                 230                 235                 240
   Gly Tyr Ala Gly Ala Asp Cys Ser Arg Glu Ile Cys Pro Val Pro Cys
                   245                 250                 255
   Ser Glu Glu His Gly Thr Cys Val Asp Gly Leu Cys Val Cys His Asp
               260                 265                 270
   Gly Phe Ala Gly Asp Asp Cys Asn Lys Pro Leu Cys Leu Asn Asn Cys
           275                 280                 285
   Tyr Asn Arg Gly Arg Cys Val Glu Asn Glu Cys Val Cys Asp Glu Gly
       290                 295                 300
   Phe Thr Gly Glu Asp Cys Ser Glu Leu Ile Cys Pro Asn Asp Cys Phe
   305                 310                 315                 320
   Asp Arg Gly Arg Cys Ile Asn Gly Thr Cys Tyr Cys Glu Glu Gly Phe
                   325                 330                 335
   Thr Gly Glu Asp Cys Gly Lys Pro Thr Cys Pro His Ala Cys His Thr
               340                 345                 350
   Gln Gly Arg Cys Glu Glu Gly Gln Cys Val Cys Asp Glu Gly Phe Ala
           355                 360                 365
   Gly Leu Asp Cys Ser Glu Lys Arg Cys Pro Ala Asp Cys His Asn Arg
       370                 375                 380
   Gly Arg Cys Val Asp Gly Arg Cys Glu Cys Asp Asp Gly Phe Thr Gly
   385                 390                 395                 400
   Ala Asp Cys Gly Glu Leu Lys Cys Pro Asn Gly Cys Ser Gly His Gly
                   405                 410                 415
   Arg Cys Val Asn Gly Gln Cys Val Cys Asp Glu Gly Tyr Thr Gly Glu
               420                 425                 430
   Asp Cys Ser Gln Leu Arg Cys Pro Asn Asp Cys His Ser Arg Gly Arg
           435                 440                 445
   Cys Val Glu Gly Lys Cys Val Cys Glu Gln Gly Phe Lys Gly Tyr Asp
       450                 455                 460
   Cys Ser Asp Met Ser Cys Pro Asn Asp Cys His Gln His Gly Arg Cys
   465                 470                 475                 480
   Val Asn Gly Met Cys Val Cys Asp Asp Gly Tyr Thr Gly Glu Asp Cys
                   485                 490                 495
   Arg Asp Arg Gln Cys Pro Arg Asp Cys Ser Asn Arg Gly Leu Cys Val
               500                 505                 510
   Asp Gly Gln Cys Val Cys Glu Asp Gly Phe Thr Gly Pro Asp Cys Ala
           515                 520                 525
   Glu Leu Ser Cys Pro Asn Asp Cys His Gly Gln Gly Arg Cys Val Asn
       530                 535                 540
   Gly Gln Cys Val Cys His Glu Gly Phe Met Gly Lys Asp Cys Lys Glu
   545                 550                 555                 560
   Gln Arg Cys Pro Ser Asp Cys His Gly Gln Gly Arg Cys Val Asp Gly
                   565                 570                 575
   Gln Cys Ile Cys His Glu Gly Phe Thr Gly Leu Asp Cys Gly Gln His
               580                 585                 590
   Ser Cys Pro Ser Asp Cys Asn Asn Leu Gly Gln Cys Val Ser Gly Arg
           595                 600                 605
   Cys Ile Cys Asn Glu Gly Tyr Ser Gly Glu Asp Cys Ser Glu Val Ser
       610                 615                 620
   Pro Pro Lys Asp Leu Val Val Thr Glu Val Thr Glu Glu Thr Val Asn
   625                 630                 635                 640
   Leu Ala Trp Asp Asn Glu Met Arg Val Thr Glu Tyr Leu Val Val Tyr
                   645                 650                 655
   Thr Pro Thr His Glu Gly Gly Leu Glu Met Gln Phe Arg Val Pro Gly
               660                 665                 670
   Asp Gln Thr Ser Thr Ile Ile Gln Glu Leu Glu Pro Gly Val Glu Tyr
           675                 680                 685
   Phe Ile Arg Val Phe Ala Ile Leu Glu Asn Lys Lys Ser Ile Pro Val
       690                 695                 700
   Ser Ala Arg Val Ala Thr Tyr Leu Pro Ala Pro Glu Gly Leu Lys Phe
   705                 710                 715                 720
   Lys Ser Ile Lys Glu Thr Ser Val Glu Val Glu Trp Asp Pro Leu Asp
                   725                 730                 735
   Ile Ala Phe Glu Thr Trp Glu Ile Ile Phe Arg Asn Met Asn Lys Glu
               740                 745                 750
   Asp Glu Gly Glu Ile Thr Lys Ser Leu Arg Arg Pro Glu Thr Ser Tyr
           755                 760                 765
   Arg Gln Thr Gly Leu Ala Pro Gly Gln Glu Tyr Glu Ile Ser Leu His
       770                 775                 780
   Ile Val Lys Asn Asn Thr Arg Gly Pro Gly Leu Lys Arg Val Thr Thr
   785                 790                 795                 800
   Thr Arg Leu Asp Ala Pro Ser Gln Ile Glu Val Lys Asp Val Thr Asp
                   805                 810                 815
   Thr Thr Ala Leu Ile Thr Trp Phe Lys Pro Leu Ala Glu Ile Asp Gly
               820                 825                 830
   Ile Glu Leu Thr Tyr Gly Ile Lys Asp Val Pro Gly Asp Arg Thr Thr
           835                 840                 845
   Ile Asp Leu Thr Glu Asp Glu Asn Gln Tyr Ser Ile Gly Asn Leu Lys
       850                 855                 860
   Pro Asp Thr Glu Tyr Glu Val Ser Leu Ile Ser Arg Arg Gly Asp Met
   865                 870                 875                 880
   Ser Ser Asn Pro Ala Lys Glu Thr Phe Thr Thr Gly Leu Asp Ala Pro
                   885                 890                 895
   Arg Asn Leu Arg Arg Val Ser Gln Thr Asp Asn Ser Ile Thr Leu Glu
               900                 905                 910
   Trp Arg Asn Gly Lys Ala Ala Ile Asp Ser Tyr Arg Ile Lys Tyr Ala
           915                 920                 925
   Pro Ile Ser Gly Gly Asp His Ala Glu Val Asp Val Pro Lys Ser Gln
       930                 935                 940
   Gln Ala Thr Thr Lys Thr Thr Leu Thr Gly Leu Arg Pro Gly Thr Glu
   945                 950                 955                 960
   Tyr Gly Ile Gly Val Ser Ala Val Lys Glu Asp Lys Glu Ser Asn Pro
                   965                 970                 975
   Ala Thr Ile Asn Ala Ala Thr Glu Leu Asp Thr Pro Lys Asp Leu Gln
               980                 985                 990
   Val Ser Glu Thr Ala Glu Thr Ser  Leu Thr Leu Leu Trp  Lys Thr Pro
           995                 1000                1005
   Leu Ala  Lys Phe Asp Arg Tyr  Arg Leu Asn Tyr Ser  Leu Pro Thr
       1010                1015                1020
   Gly Gln  Trp Val Gly Val Gln  Leu Pro Arg Asn Thr  Thr Ser Tyr
       1025                1030                1035
   Val Leu  Arg Gly Leu Glu Pro  Gly Gln Glu Tyr Asn  Val Leu Leu
       1040                1045                1050
   Thr Ala  Glu Lys Gly Arg His  Lys Ser Lys Pro Ala  Arg Val Lys
       1055                1060                1065
   Ala Ser  Thr Glu Gln Ala Pro  Glu Leu Glu Asn Leu  Thr Val Thr
       1070                1075                1080
   Glu Val  Gly Trp Asp Gly Leu  Arg Leu Asn Trp Thr  Ala Ala Asp
       1085                1090                1095
   Gln Ala  Tyr Glu His Phe Ile  Ile Gln Val Gln Glu  Ala Asn Lys
       1100                1105                1110
   Val Glu  Ala Ala Arg Asn Leu  Thr Val Pro Gly Ser  Leu Arg Ala
       1115                 1120                 1125
   Val Asp  Ile Pro Gly Leu Lys  Ala Ala Thr Pro Tyr  Thr Val Ser
       1130                 1135                 1140
   Ile Tyr  Gly Val Ile Gln Gly  Tyr Arg Thr Pro Val  Leu Ser Ala
       1145                 1150                 1155
   Glu Ala  Ser Thr Gly Glu Thr  Pro Asn Leu Gly Glu  Val Val Val
       1160                 1165                 1170
   Ala Glu  Val Gly Trp Asp Ala  Leu Lys Leu Asn Trp  Thr Ala Pro
       1175                 1180                 1185
   Glu Gly  Ala Tyr Glu Tyr Phe  Phe Ile Gln Val Gln  Glu Ala Asp
       1190                 1195                 1200
   Thr Val  Glu Ala Ala Gln Asn  Leu Thr Val Pro Gly  Gly Leu Arg
       1205                 1210                 1215
   Ser Thr  Asp Leu Pro Gly Leu  Lys Ala Ala Thr His  Tyr Thr Ile
       1220                 1225                 1230
   Thr Ile  Arg Gly Val Thr Gln  Asp Phe Ser Thr Thr  Pro Leu Ser
       1235                 1240                 1245
   Val Glu  Val Leu Thr Glu Glu  Val Pro Asp Met Gly  Asn Leu Thr
       1250                 1255                 1260
   Val Thr  Glu Val Ser Trp Asp  Ala Leu Arg Leu Asn  Trp Thr Thr
       1265                 1270                 1275
   Pro Asp  Gly Thr Tyr Asp Gln  Phe Thr Ile Gln Val  Gln Glu Ala
       1280                 1285                 1290
   Asp Gln  Val Glu Glu Ala His  Asn Leu Thr Val Pro  Gly Ser Leu
       1295                 1300                 1305
   Arg Ser  Met Glu Ile Pro Gly  Leu Arg Ala Gly Thr  Pro Tyr Thr
       1310                 1315                 1320
   Val Thr  Leu His Gly Glu Val  Arg Gly His Ser Thr  Arg Pro Leu
       1325                 1330                 1335
   Ala Val  Glu Val Val Thr Glu  Asp Leu Pro Gln Leu  Gly Asp Leu
   1340 1345 1350
   Ala Val Ser Glu Val Gly Trp Asp Gly Leu Arg Leu Asn Trp Thr
   1355 1360 1365
   Ala Ala Asp Asn Ala Tyr Glu His Phe Val Ile Gln Val Gln Glu
   1370 1375 1380
   Val Asn Lys Val Glu Ala Ala Gln Asn Leu Thr Leu Pro Gly Ser
   1385 1390 1395
   Leu Arg Ala Val Asp Ile Pro Gly Leu Glu Ala Ala Thr Pro Tyr
   1400 1405 1410
   Arg Val Ser Ile Tyr Gly Val Ile Arg Gly Tyr Arg Thr Pro Val
   1415 1420 1425
   Leu Ser Ala Glu Ala Ser Thr Ala Lys Glu Pro Glu Ile Gly Asn
   1430 1435 1440
   Leu Asn Val Ser Asp Ile Thr Pro Glu Ser Phe Asn Leu Ser Trp
   1445 1450 1455
   Met Ala Thr Asp Gly Ile Phe Glu Thr Phe Thr Ile Glu Ile Ile
   1460 1465 1470
   Asp Ser Asn Arg Leu Leu Glu Thr Val Glu Tyr Asn Ile Ser Gly
   1475 1480 1485
   Ala Glu Arg Thr Ala His Ile Ser Gly Leu Pro Pro Ser Thr Asp
   1490 1495 1500
   Phe Ile Val Tyr Leu Ser Gly Leu Ala Pro Ser Ile Arg Thr Lys
   1505 1510 1515
   Thr Ile Ser Ala Thr Ala Thr Thr Glu Ala Leu Pro Leu Leu Glu
   1520 1525 1530
   Asn Leu Thr Ile Ser Asp Ile Asn Pro Tyr Gly Phe Thr Val Ser
   1535 1540 1545
   Trp Met Ala Ser Glu Asn Ala Phe Asp Ser Phe Leu Val Thr Val
   1550 1555 1560
   Val Asp Ser Gly Lys Leu Leu Asp Pro Gln Glu Phe Thr Leu Ser
   1565 1570 1575
   Gly Thr  Gln Arg Lys Leu Glu  Leu Arg Gly Leu Ile  Thr Gly Ile
       1580                 1585                 1590
   Gly Tyr  Glu Val Met Val Ser  Gly Phe Thr Gln Gly  His Gln Thr
       1595                 1600                 1605
   Lys Pro  Leu Arg Ala Glu Ile  Val Thr Glu Ala Glu  Pro Glu Val
       1610                 1615                 1620
   Asp Asn  Leu Leu Val Ser Asp  Ala Thr Pro Asp Gly  Phe Arg Leu
       1625                 1630                 1635
   Ser Trp  Thr Ala Asp Glu Gly  Val Phe Asp Asn Phe  Val Leu Lys
       1640                 1645                 1650
   Ile Arg  Asp Thr Lys Lys Gln  Ser Glu Pro Leu Glu  Ile Thr Leu
       1655                 1660                 1665
   Leu Ala  Pro Glu Arg Thr Arg  Asp Leu Thr Gly Leu  Arg Glu Ala
       1670                 1675                 1680
   Thr Glu  Tyr Glu Ile Glu Leu  Tyr Gly Ile Ser Lys  Gly Arg Arg
       1685                 1690                 1695
   Ser Gln  Thr Val Ser Ala Ile  Ala Thr Thr Ala Met  Gly Ser Pro
       1700                 1705                 1710
   Lys Glu  Val Ile Phe Ser Asp  Ile Thr Glu Asn Ser  Ala Thr Val
       1715                 1720                 1725
   Ser Trp  Arg Ala Pro Thr Ala  Gln Val Glu Ser Phe  Arg Ile Thr
       1730                 1735                 1740
   Tyr Val  Pro Ile Thr Gly Gly  Thr Pro Ser Met Val  Thr Val Asp
       1745                 1750                 1755
   Gly Thr  Lys Thr Gln Thr Arg  Leu Val Lys Leu Ile  Pro Gly Val
       1760                 1765                 1770
   Glu Tyr  Leu Val Ser Ile Ile  Ala Met Lys Gly Phe  Glu Glu Ser
       1775                 1780                 1785
   Glu Pro  Val Ser Gly Ser Phe  Thr Thr Ala Leu Asp  Gly Pro Ser
       1790                 1795                 1800
   Gly Leu  Val Thr Ala Asn Ile  Thr Asp Ser Glu Ala  Leu Ala Arg
       1805                 1810                 1815
   Trp Gln  Pro Ala Ile Ala Thr  Val Asp Ser Tyr Val  Ile Ser Tyr
       1820                 1825                 1830
   Thr Gly  Glu Lys Val Pro Glu  Ile Thr Arg Thr Val  Ser Gly Asn
       1835                 1840                 1845
   Thr Val  Glu Tyr Ala Leu Thr  Asp Leu Glu Pro Ala  Thr Glu Tyr
       1850                 1855                 1860
   Thr Leu  Arg Ile Phe Ala Glu  Lys Gly Pro Gln Lys  Ser Ser Thr
       1865                 1870                 1875
   Ile Thr  Ala Lys Phe Thr Thr  Asp Leu Asp Ser Pro  Arg Asp Leu
       1880                 1885                 1890
   Thr Ala  Thr Glu Val Gln Ser  Glu Thr Ala Leu Leu  Thr Trp Arg
       1895                 1900                 1905
   Pro Pro  Arg Ala Ser Val Thr  Gly Tyr Leu Leu Val  Tyr Glu Ser
       1910                 1915                 1920
   Val Asp  Gly Thr Val Lys Glu  Val Ile Val Gly Pro  Asp Thr Thr
       1925                 1930                 1935
   Ser Tyr  Ser Leu Ala Asp Leu  Ser Pro Ser Thr His  Tyr Thr Ala
       1940                 1945                 1950
   Lys Ile  Gln Ala Leu Asn Gly  Pro Leu Arg Ser Asn  Met Ile Gln
       1955                 1960                 1965
   Thr Ile  Phe Thr Thr Ile Gly  Leu Leu Tyr Pro Phe  Pro Lys Asp
       1970                 1975                 1980
   Cys Ser  Gln Ala Met Leu Asn  Gly Asp Thr Thr Ser  Gly Leu Tyr
       1985                 1990                 1995
   Thr Ile  Tyr Leu Asn Gly Asp  Lys Ala Gln Ala Leu  Glu Val Phe
       2000                 2005                 2010
   Cys Asp  Met Thr Ser Asp Gly  Gly Gly Trp Ile Val  Phe Leu Arg
       2015                 2020                 2025
   Arg Lys  Asn Gly Arg Glu Asn  Phe Tyr Gln Asn Trp  Lys Ala Tyr
       2030                 2035                 2040
   Ala Ala  Gly Phe Gly Asp Arg  Arg Glu Glu Phe Trp  Leu Gly Leu
       2045                 2050                 2055
   Asp Asn  Leu Asn Lys Ile Thr  Ala Gln Gly Gln Tyr  Glu Leu Arg
       2060                 2065                 2070
   Val Asp  Leu Arg Asp His Gly  Glu Thr Ala Phe Ala  Val Tyr Asp
       2075                 2080                 2085
   Lys Phe  Ser Val Gly Asp Ala  Lys Thr Arg Tyr Lys  Leu Lys Val
       2090                 2095                 2100
   Glu Gly  Tyr Ser Gly Thr Ala  Gly Asp Ser Met Ala  Tyr His Asn
       2105                 2110                 2115
   Gly Arg  Ser Phe Ser Thr Phe  Asp Lys Asp Thr Asp  Ser Ala Ile
       2120                 2125                 2130
   Thr Asn  Cys Ala Leu Ser Tyr  Lys Gly Ala Phe Trp  Tyr Arg Asn
       2135                 2140                 2145
   Cys His  Arg Val Asn Leu Met  Gly Arg Tyr Gly Asp  Asn Asn His
       2150                 2155                 2160
   Ser Gln  Gly Val Asn Trp Phe  His Trp Lys Gly His  Glu His Ser
       2165                 2170                 2175
   Ile Gln  Phe Ala Glu Met Lys  Leu Arg Pro Ser Asn  Phe Arg Asn
       2180                 2185                 2190
   Leu Glu  Gly Arg Arg Lys Arg  Ala
       2195                 2200

## Description

L'invention concerne le diagnostic et le suivi d'une hypertension artérielle pulmonaire.

### Etat de la technique :

L'hypertension artérielle pulmonaire (HTAP) est une pathologie rare responsable de la survenue d'une décompensation cardiaque droite pouvant aboutir au décès. L'HTAP est définie par la mise en évidence par cathétérisme droit d'une pression artérielle pulmonaire moyenne supérieure ou égale à 25mmHg au repos ou à 30mmHg à l'effort en l'absence d'élévation de la pression capillaire pulmonaire (Rubin, 1997). La survenue d'une HTAP est la conséquence d'une obstruction chronique des petites artères pulmonaires secondaire à la prolifération de cellules endothéliales, de cellules musculaires lisses vasculaires et de fibroblastes (Dorfmuller et al, 2003). En particulier, au cours de l'HTAP sévère, il se forme une couche de myofibroblastes et de matrice extra-cellulaire qui se localise entre l'endothélium et la limitante élastique interne, appelée neo-intima, qui est caractéristique de cette affection.

L'HTAP peut survenir dans l'évolution de pathologies à composante auto-immune que sont les connectivites, en particulier la sclérodermie systémique (Hachulla et al, 2005), le syndrome de Sharp et le lupus érythémateux systémique. De plus, au cours de l'HTAP idiopathique, on trouve de temps à autres des stigmates d'auto-immunité, à savoir des anticorps anti-nucléaires ou des anticorps anti-thyroglobuline.

La présence d'anticorps anti-cellules endothéliales (Tamby et al, 2005) et d'anticorps antifibroblastes (Tamby et al, 2006) a été rapportée au cours de l'HTAP idiopathique ou associée à la sclérodermie systémique. Cependant la valeur prédictive de ces anticorps dans la survenue de l'HTAP n'a pas été étudiée et le rôle potentiel des phénomènes autoimmuns dans la pathogénie de l'HTAP idiopathique reste incertain (Mouthon et al, 2005). Dans la plupart des cas, l'HTAP est dépistée lorsque le patient présente une dyspnée de stade III ou IV. Lorsque le patient est suivi pour une maladie chronique comme la sclérodermie systémique, l'HTAP est dépistée par une échographie cardiaque annuelle. Cependant, un test simple et fiable de dépistage d'une HTAP fait toujours défaut, et serait précieux pour un diagnostic le plus précoce possible, qui permettrait de mettre en place rapidement des stratégies thérapeutiques pour améliorer l'état du patient et ses chances de survie.

### Résumé de l'invention :

L'invention fournit maintenant un procédé *in vitro* de détection d'une HTAP, ou d'un risque de développer une HTAP, comprenant la détermination de la présence et/ou de la quantité d'anticorps anti-Ténascine C (TN-C) dans un échantillon biologique provenant d'un patient, la présence d'anticorps anti-TN-C étant indicatrice d'une HTAP ou d'un risque de développer une HTAP.

De préférence, la présence d'anticorps anti-Ténascine C dans l'échantillon biologique est comparée à une valeur contrôle, la présence d'anticorps anti-Ténascine C en une quantité supérieure à la valeur contrôle étant indicatrice d'une HTAP ou d'un risque de développer une HTAP.

Un autre objet de l'invention est un *procédé in vitro* de pronostic ou de suivi d'une HTAP, comprenant la détermination de la présence et/ou de la quantité d'anticorps anti-TN-C dans un échantillon biologique provenant d'un patient, à différents temps, l'augmentation de la quantité d'anticorps anti-TN-C au cours du temps étant indicatif d'une aggravation de l'HTAP.

Un autre objet de l'invention est un *procédé in vitro* d'évaluation de l'efficacité d'un traitement envers une HTAP, comprenant la détermination de la présence et/ou de la quantité d'anticorps anti-TN-C dans un échantillon biologique provenant d'un patient, à différents temps avant, au cours ou après le traitement, la diminution de la quantité d'anticorps anti-TN-C au cours du temps étant indicatif d'une amélioration de l'HTAP.

### Description détaillée de l'invention :

La TN-C est exprimée à l'intérieur et autour des vaisseaux sanguins dans le poumon foetal (Rettig et al, 1994), mais elle n'est plus exprimée ensuite dans les artères pulmonaires adultes normales (Jones et al, 1996). Par ailleurs, la perte de signalisation via BMPRII, à l'origine d'un défaut de cellules T régulatrices pouvant prédisposer à la survenue d'une HTAP (Nicolls et al, 2005), peut également induire l'expression de la TN-C *in vivo* et sur des cellules vasculaires en culture (Ihida-Stansbury et al, 2006). Sur cette base, les inventeurs ont émis l'hypothèse que des patients ayant une HTAP pourraient développer une réponse immune dirigée contre la TN-C. Aussi ont-ils décidé de rechercher des anticorps anti-TN-C dans le sérum de patients atteints d'HTAP.

Les inventeurs ont ainsi pu mettre en évidence une corrélation entre la survenue d'une HTAP et la production d'anticorps anti-TN-C. Sur cette base, ils proposent un procédé *in vitro* de diagnostic ou de pronostic d'une HTAP, ou d'un risque de développer une HTAP, comprenant la détermination de la présence et/ou de la quantité d'anticorps anti-TN-C dans un échantillon biologique provenant d'un patient. Les anticorps anti-TN-C détectés sont de préférence des immunoglobulines G (IgG).

### Définitions

La Ténascine C (ou TN-C) est une glycoprotéine de la matrice extracellulaire. Elle est également connue sous le nom de hexabrachion ou cytotactine. Une séquence de la TN-C humaine est rapportée en annexe (SEQ ID n°1).

Le terme "échantillon biologique" se réfère à tout échantillon biologique provenant d'un patient. Des exemples d'échantillons incluent des liquides biologiques, des biopsies tissulaires. De manière préférentielle, l'échantillon peut être du sang, du sérum, de la salive, de l'urine, du sperme. De manière davantage préférée, l'échantillon biologique est un échantillon de sang ou de sérum.

Le terme "patient" se réfère à tout sujet susceptible d'être testé. De préférence il s'agit d'un humain, mais le terme inclut tout autre mammifère, tel que des chiens, chats, rongeurs, bétail, chevaux, singes etc. Le patient peut être testé quel que soit son sexe ou son âge. Le patient peut être un sujet à risque, être asymptomatique ou présenter des signes précoces ou avancés d'une HTAP. Par exemple le patient peut être un sujet prédisposé à développer une HTAP, en particulier un sujet portant une ou plusieurs mutations dans le gène codant pour BMPRII.

Le terme "diagnostic" signifie l'identification de la pathologie ou l'évaluation de l'état de sévérité de la pathologie.

Le terme « pronostic» signifie l'évaluation du risque d'aggravation, et de ses conséquences.

Le terme « valeur contrôle » se réfère à une valeur basale correspondant à la moyenne des valeurs obtenues avec l'échantillon biologique de sujets sains, non affectés par une HTAP ou une maladie susceptible d'entraîner une HTAP. Il peut s'agir d'une valeur statistique de référence.

Pour évaluer l'évolution de la pathologie, il peut être utile de tester un patient et de contrôler l'effet d'un traitement ou l'évolution de la pathologie, en testant de nouveau le patient, par exemple à plusieurs mois d'intervalle. Dans ce cas, les résultats du second test sont comparés aux résultats du premier test, ainsi que souvent à la valeur dite « contrôle ». Une quantité d'anticorps anti-TN-C « supérieure à la valeur contrôle » signifie généralement une augmentation statistiquement significative, par exemple d'au moins deux déviations standards au dessus de la moyenne des densités optiques des réactivités IgG de l'ensemble des sujets sains.

Par "antigène de capture", on entend un antigène, de préférence fixé sur une phase solide, qui est capable de retenir l'anticorps anti-TN-C présent dans un échantillon biologique, par liaison affine. L'antigène de capture peut être marqué.

Le terme "marqué" se réfère aussi bien à un marquage direct (par le biais d'enzymes, radioisotopes, fluorochromes, composés luminescents, etc...) qu'à un marquage indirect (par exemple, par le biais d'anticorps eux-mêmes marqués de manière directe ou à l'aide de réactifs d'une "paire d'affinité " marquée, telle que, mais non exclusivement, la paire avidine marquée-biotine, etc..).

### Dosage des anticorps :

L'échantillon biologique est de préférence un échantillon de sérum, dilué au 1/100^{ème}, ou plus, par exemple au 1/200^{ème} ou 1/400^{ème}.

De manière avantageuse, la quantité d'anticorps anti-TN-C peut être déterminée par un immunoessai.

L'échantillon biologique peut être éventuellement traité dans une étape préalable, ou mis directement en présence d'au moins un antigène de capture.

Le procédé selon l'invention peut être réalisé selon divers formats bien connus de l'homme du métier: en phase solide ou en phase homogène; en un temps ou en deux temps; en méthode compétitive, à titre d'exemples non limitatifs.

Selon un mode de réalisation préféré, l'antigène de capture est immobilisé sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque EstaporTM), ou encore des tubes à essai en polystyrène ou polypropylène, etc.

Un format d'immunoessai de détection des anticorps par compétition est également possible. D'autres modalités d'immunoessai sont encore envisageables et bien connues de l'homme du métier.

Dosages ELISA, radioimmunoessais, ou toute autre technique de détection peuvent être mis en oeuvre pour révéler la présence des complexes antigènes-anticorps formés.

Selon un mode de réalisation préféré, le procédé de l'invention comprend la mise en contact d'un échantillon biologique avec une protéine comprenant le fragment d'acides aminés 181 à 290 de la séquence TN-C humaine telle que représentée en SEQ ID NO : 1.

Dans un exemple particulier, l'antigène de capture, qui peut être une protéine comprenant le fragment d'acides aminés 181 à 290 de la séquence TN-C humaine, peut être couplé à une glutathion S transférase (GST), avant d'être déposé sur une microplaque.

Des échantillons de sérum à tester, préalablement dilués au 1/100ème, sont mis à incuber sur la microplaque. Après lavage, des anticorps anti-Fc γ humain marqués (par exemple avec une phosphatase alcaline) sont ajoutés, les complexes étant révélés (par exemple par ajout d'un substrat de la phosphatase dont le clivage peut être détecté par lecture de l'absorbance).

### Patients visés :

Les patients visés sont ceux susceptibles de développer une HTAP.

Il peut s'agir d'un patient qui souffre d'une connectivite, telle qu'une sclérodermie systémique, d'un syndrome de Sharp (qui est une connectivite mixte), d'un lupus érythémateux systémique.

Le patient peut également souffrir d'une HTAP idiopathique ou familiale.

Plus généralement, tout patient atteint d'une maladie vasculaire pulmonaire peut être avantageusement soumis au procédé de détection d'une HTAP tel que défini dans l'invention.

Par ailleurs, l'HTAP détectée peut être aussi une hypertension porto-pulmonaire (c'est-à-dire une HTAP associée à une hypertension portale), ou être associée à une cardiopathie congénitale, ou à une infection par le virus de l'immunodéficience humaine (VIH), ou encore être une hypertension pulmonaire post-embolique, compliquant l'évolution d'une bronchite chronique obstructive ou d'une cardiopathie cyanogène.

D'autres patients visés sont ceux exposés à certains médicaments coupe-faim comme la fenfluramine dont la prescription peut contribuer à la survenue d'une HTAP.

D'autres personnes susceptibles de bénéficier de ce type de test sont celles porteuses d'une mutation dans le gène codant pour BMPRII et qui éventuellement ne présentent pas HTAP détectable à l'échographie, de façon à dépister les individus susceptibles de développer ultérieurement une HTAP.

### Evaluation de l'efficacité d'un traitement

Un autre objet de l'invention est un procédé *in vitro* d'évaluation de l'efficacité d'un traitement envers une HTAP, comprenant la détermination de la présence et/ou de la quantité d'anticorps anti-TN-C dans un échantillon biologique provenant d'un patient, à différents temps avant, au cours ou après le traitement, la diminution de la quantité d'anticorps anti-TN-C au cours du temps étant indicatif d'une amélioration de l'HTAP.

Le traitement classique actuel de l'HTAP associe un traitement symptomatique et un traitement vasodilatateur. Le traitement symptomatique associe des anti-coagulants, une oxygénothérapie et des diurétiques. Le traitement vasodilatateur repose sur les molécules suivantes : bloqueurs de canaux calcium, époprosténol (prostacycline) prescrit par voie intraveineuse en perfusion continue, les inhibiteurs des récepteurs de l'endothéline, sélectifs ou non, en particulier le bosentan, le sytaxentan et l'ambrysentan, les inhibiteurs des phosphodiestérases de type 5 en particulier le sildénafil, l'ensemble de ces médicaments étant administrés par voie orale, et l'iloprost inhalé, analogue de la prostacycline administré par voie inhalée. Ces traitements peuvent être éventuellement combinés. En cas d'échec de ces thérapeutiques, une transplantation pulmonaire ou coeur-poumons peut être proposée.

Les figures et exemples suivants illustrent l'invention sans en limiter la portée.

### Légende des figures :

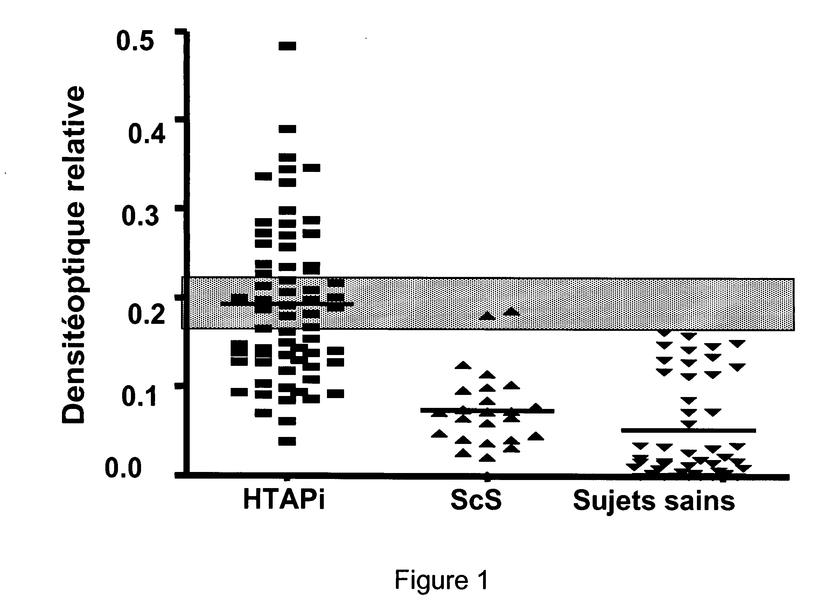

La figure 1 est un graphe montrant la détection des anticorps anti-TN-C par dosage ELISA. Les IgG sériques des patients atteints d'HTAP idiopathique, des patients atteints de sclérodermie systémique et des sujets sains appariés pour le sexe et l'âge, ont été testées vis à vis d'un fragment de TN-C recombinante, à une dilution de 1/100ème. Les limites inférieure et supérieure de la zone en pointillé représentent les seuils définis par deux et trois fois l'écart-type au dessus de la moyenne des densités optiques obtenues chez les sujets sains. Les différences significatives entre les groupes de malades et les sujets sains sont estimées à l'aide d'un test de rang de Mann Whitney et sont indiquées par :
   *: p <0,01
   **: p<0,001.

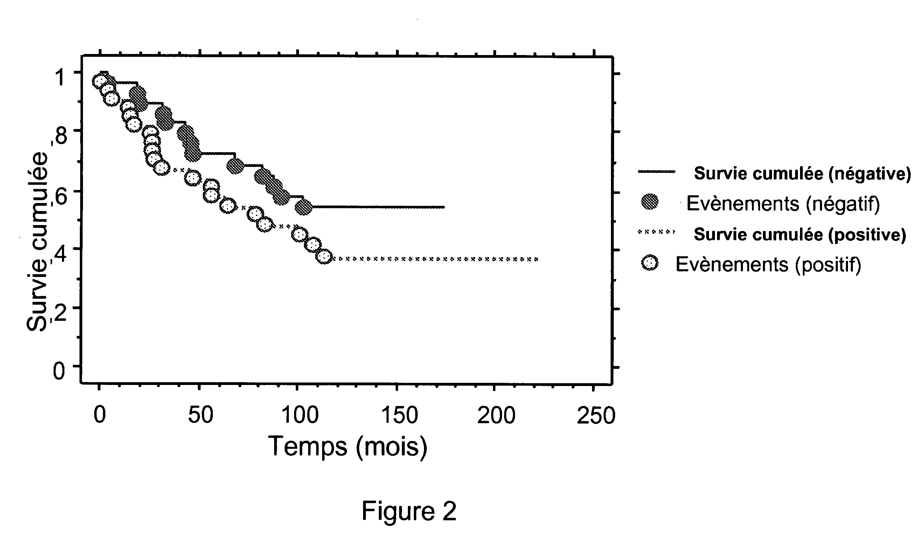

La figure 2 représente des courbes de survie selon Kaplan et Meier en fonction de la présence ou de l'absence d'anticorps anti-TN-C. En abscisse, le temps en mois ; en ordonnée, la survie cumulée en pourcentage.

### Exemple : Détection d'anticorps anti-Ténascine C chez des patients atteints d'hypertension artérielle pulmonaire.

### Matériels et méthodes :

### Patients

L'HTAP a été dépistée par la mise en évidence par échographie cardiaque trans-thoracique d'une pression artérielle pulmonaire systolique supérieure à 40mmHg. Dans tous les cas, l'HTAP a été confirmée par la réalisation d'un cathétérisme droit et la mise en évidence d'une pression artérielle pulmonaire moyenne supérieure ou égale à 25 mmHg au repos et à 30 mmHg à l'effort. Par convention, l'HTAP était qualifiée d'idiopathique si le patient ne montrait aucune pathologie associée, l'HTAP pouvant alors correspondre à une HTAP sporadique, familiale ou associée à une exposition à la fenfluramine. 91 patients ont été inclus dans l'étude comprenant 66 (72,5%) patients ayant une HTAP idiopathique (IPAH) et 25 patients ayant une sclérodermie systémique répondant aux critères de l'American College of Rheumatology (ACR) et/ou aux critères de LeRoy et Medsger (Masi et al, 1980 ; LeRoy et al, 2001).

Tous les patients ayant une sclérodermie systémique diffuse sans HTAP avaient une atteinte interstitielle pulmonaire mise en évidence par un scanner thoracique haute résolution et une capacité vitale inférieure à 80% de la valeur prédite et/ou un coefficient de transfert du monoxyde de carbone (DLCO) inférieure à 75% de la valeur prédite. Aucun des patients ne recevait des corticoïdes ou d'immunosuppresseurs au moment des prélèvements, aucun d'entre eux n'avait de tumeur solide ou une autre connectivite associée. 46 sujets sains appariés pour le sexe et l'âge ont été utilisés comme contrôles.

### Dosage ELISA

La Ténascine C (TN-C) a été obtenue auprès de la société Abnova (Abnova Corporation, Taipei city, Taïwan). L'antigène utilisé était constitué du fragment 181 à 290 de la TN-C (SEQ ID n°1 ), couplé à un motif GST. La TN-C a été diluée dans un tampon de bicarbonate et déposée sur des plaques 96 puits (Maxisorb, NalgeNunc Int. Rochester, NY, USA) à une concentration finale de 4µg/mL à 4°C. Les sérums de patients et de sujets sains ont été dilués au 1/100^{ème} dans un tampon phosphate (PBS) à 1% d'albumine et incubés une heure à 37°C. Après un lavage, des anticorps de lapin anti-Fcy humain conjugués à de la phosphatase alcaline (Dakocytomation, Golstrup, Denmark), ont été ajoutés et incubés pendant une heure à température ambiante. Les réactivités ont été révélées par ajout de p-nitrophenylphosphate à 0,05M dans un tampon de carbonate de magnésium (pH 9,8) et l'absorbance à 405nm a été déterminée en utilisant un lecteur de plaques ELISA (Fusion, Packard BioScience, Meriden, CT, USA). Afin de tenir compte de la variabilité entre les puits, la densité optique d'un sérum de référence était arbitrairement définie comme 100% de l'activité anti-TN C. Les résultats des échantillons testés ont été calculés à partir de la moyenne de l'absorbance de puits dupliqués et exprimée comme un pourcentage de cette valeur de référence. Tous les échantillons ont été testés en double.

### Analyses statistiques

Toutes les analyses statistiques ont été effectuées en utilisant le logiciel Systat (version 11.0 Systat Software Inc, Point Richmond, CA, USA). Un test de Mann-Whitney a été utilisé pour comparer les densités optiques relatives des différents groupes. Des valeurs de P inférieures à 0,05 étaient considérées comme statistiquement significatives. La survie a été calculée par la méthode de Kaplan et Meier (Kaplan and Meier, 1958).

### Résultats :

Les réactivités des IgG des patients atteints d'HTAP idiopathique, des patients atteints de sclérodermie systémique avec ou sans HTAP et des sujets contrôles vis à vis de la TN-C ont été étudiés par ELISA. En utilisant un seuil défini par deux déviations standard au-dessus de la moyenne des densités optiques des réactivités IgG de l'ensemble des sujets sains, 36/66 (54,5%) patients ayant une HTAP idiopathique et 2/25 (8%) des patients sclérodermiques avaient des IgG anti-TN-C. Aucun des sujets sains n'avait d'IgG anti-TN-C (Figure 1). Lorsque le seuil était déplacé à trois déviations standard au dessus de la moyenne des réactivités IgG des individus sains, 12/66 (18,1 %) des patients ayant une HTAP idiopathique avait des IgG anti-TN C et aucun patient sclérodermique n'avait d'IgG anti-TN C. Les réactivités des IgG sériques des anticorps anti-TN C de patients ayant une HTAP idiopathique étaient significativement plus élevées que celles des patients sclérodermiques (p<0,001), et que celles des sujets sains (p<0,001). De la même façon, les réactivités des IgG sériques des anticorps anti-TN C de patients sclérodermiques étaient significativement plus élevées que celles des individus sains (p=0,021) (Figure 1).

Il n'a pas été mis en évidence de différence significative dans la présentation clinique et les données de l'échographie cardiaque, du cathétérisme droit et du test de marche de 6 minutes entre les deux groupes de patients. La survie était diminuée dans le groupe des malades ayant des anticorps anti-TNC comparativement aux malades qui n'avaient pas d'anticorps anti-TN C sans toutefois que cette différence ne soit ici significative (p=0,17).

L'apparition d'une réponse immune dirigée contre la TN C pourrait résulter des mêmes mécanismes que ceux conduisant à l'induction de l'expression de la TN C et à la prolifération des cellules musculaires lisses. La présence d'anticorps anti-TN C serait donc corrélée à l'apparition d'un remodelage vasculaire, constituant un marqueur de la survenue d'une HTAP.

### Références bibliographiques

- Dorfinuller et al, 2003, Eur Respir J, 22(2) :358-63
- Hachulla et al, 2005, Arthritis Rheum 52(12) :3792-3800
- Ihida-Stansbury et al, 2006, Am J Physiol Lung Cell Mol Physiol 291(4):L694-702
- Jones et al, 1996, Circ Res 79(6) :1131-42
- Kaplan and Meier, 1958, J Am Stat Assoc 53:457-81
- LeRoy et al, 2001, J Rheumatol 28(7):1573-76
- Masi et al, 1980, Arthr Rheum 23 :581-90
- Mouthon et al, 2005, Eur Respir J 26(6) :986-8
- Nicolls et al, 2005, Eur Respir J 26(6) 1110-8
- Rettig et al, 1994, J Cell Sci;107 (Pt 2):487-97
- Rubin, 1997, N Engl J Med, 336(2) :111-7
- Tamby et al, 2005, Thorax 60(9):765-72
- Tamby et al, 2006, Eur Respir J 28(4):799-807

## Revendications

1. Procédé *in vitro* de détection d'une hypertension artérielle pulmonaire (HTAP), ou d'un risque de développer une HTAP, comprenant la détermination de la présence et/ou de la quantité d'anticorps anti-Ténascine C dans un échantillon biologique provenant d'un patient, la présence d'anticorps anti-Ténascine C étant indicatrice d'une HTAP ou d'un risque de développer une HTAP.

2. Procédé selon la revendication 1, dans lequel l'échantillon biologique est un échantillon de sang ou de sérum.

3. Procédé selon la revendication 1 ou 2, dans lequel la présence d'anticorps anti-Ténascine C dans l'échantillon biologique est comparée à une valeur contrôle, la présence d'anticorps anti-Ténascine C en une quantité supérieure à la valeur contrôle étant indicatrice d'une HTAP ou d'un risque de développer une HTAP.

4. Procédé selon l'une des revendications 1 à 3, dans lequel la quantité d'anticorps anti-Ténascine C est déterminée par un immunoessai.

5. Procédé selon la revendication 4, dans lequel l'immunoessai est un dosage ELISA.

6. Procédé selon la revendication 4 ou 5, comprenant la mise en contact d'un échantillon biologique avec une protéine comprenant le fragment d'acides aminés 181 à 290 de la séquence Ténascine C humaine telle que représentée en SEQ ID NO :1.

7. Procédé selon l'une des revendications 1 à 6, dans lequel le patient est un humain.

8. Procédé selon l'une des revendications 1 à 7, dans lequel le patient souffre d'une sclérodermie systémique.

9. Procédé selon l'une des revendications 1 à 7, dans lequel le patient souffre d'un syndrome de Sharp.

10. Procédé selon l'une des revendications 1 à 7, dans lequel le patient souffre d'un lupus érythémateux systémique.

11. Procédé selon l'une des revendications 1 à 7, dans lequel le patient souffre d'une HTAP idiopathique.

12. Procédé selon l'une des revendications 1 à 7, dans lequel l'HTAP est associée à une hypertension portale, à une cardiopathie congénitale, ou à une infection par le virus de l'immunodéficience humaine (VIH), ou est une hypertension pulmonaire post-embolique.

13. Procédé selon l'une des revendications 1 à 7, dans lequel le patient est un sujet prédisposé à développer une HTAP, ledit sujet étant de préférence porteur d'une ou plusieurs mutation(s) dans le gène codant pour BMPRII.

14. Procédé *in vitro* de pronostic ou de suivi d'une HTAP, comprenant la détermination de la présence et/ou de la quantité d'anticorps anti-Ténascine C dans un échantillon biologique provenant d'un patient, à différents temps, l'augmentation de la quantité d'anticorps anti-Ténascine C au cours du temps étant indicatif d'une aggravation de l'HTAP.

15. Procédé *in vitro* d'évaluation de l'efficacité d'un traitement envers une HTAP, comprenant la détermination de la présence et/ou de la quantité d'anticorps anti-Ténascine C dans un échantillon biologique provenant d'un patient, à différents temps avant, au cours ou après le traitement, la diminution de la quantité d'anticorps anti-Ténascine C au cours du temps étant indicatif d'une amélioration de l'HTAP.

## Claims

1. An *in vitro* method for detecting pulmonary arterial hypertension PAH), or a risk of developing PAH, which comprises determining the presence and/or the amount of anti-tenascin C antibodies in a biological sample from a patient, the presence of anti-tenascin C antibodies being indicative of PAH or a risk of developing PAH.

2. The method according to claim 1, wherein the biological sample is a blood or serum sample.

3. The method according to claim 1 or 2, wherein the presence of anti-tenascin C antibodies in the biological sample is compared with a control value, the presence of anti-tenascin C antibodies in an amount greater than the control value being indicative of PAH or of a risk of developing PAH.

4. The method according to one of claims 1 to 3, wherein the amount of anti-tenascin C antibodies is determined by means of an immunoassay.

5. The method according to claim 4, wherein the immunoassay is an ELISA assay.

6. The method according to claim 4 or 5, which comprises contacting a biological sample with a protein comprising amino acid fragment 181 to 290 of the human tenascin C sequence as represented in SEQ ID No. 1.

7. The method according to one of claims 1 to 6, wherein the patient is a human being.

8. The method according to one of claims 1 to 7, wherein the patient suffers from systemic scleroderma.

9. The method according to one of claims 1 to 7, wherein the patient suffers from Sharp's syndrome.

10. The method according to one of claims 1 to 7, wherein the patient suffers from erythematosus systemic lupus.

11. The method according to one of claims 1 to 7, wherein the patient suffers from idiopathic PAH.

12. The method according to one of claims 1 to 7, wherein the PAH is associated with portal hypertension, with congenital heart disease, or with a human immunodeficiency virus (HIV) infection, or is post-embolic pulmonary hypertension.

13. The method according to one of claims 1 to 7, wherein the patient is an individual predisposed to developing PAH, said individual being preferably carrier of one or more mutation(s) in the BMPRII encoding gene.

14. An *in vitro* method for the prognosis or the follow-up of PAH, which comprises determining the presence and/or the amount of anti-tenascin C antibodies in a biological sample from a patient, at various times, an increase in the amount of anti-tenascin C antibodies over time being indicative of a worsening of the PAH.

15. An *in vitro* method for evaluating the efficacy of a treatment for PAH, which comprises determining the presence and/or the amount of anti-tenascin C antibodies in a biological sample from a patient, at various times before, during or after the treatment, a decrease in the amount of anti-tenascin C antibodies over time being indicative of an improvement in the PAH.

## Patentansprüche

1. *In vitro* Verfahren zum Nachweis von pulmonal arterieller Hypertonie (PAH) oder einem Risiko der Entwicklung von PAH, umfassend die Bestimmung des Vorhandenseins und/oder der Menge an Anti-Tenascin C-Antikörpern in einer von einem Patienten stammenden biologischen Probe, wobei das Vorhandensein von Anti-Tenascin C-Antikörpern auf PAH oder auf ein Risiko der Entwicklung von PAH hinweist.

2. Verfahren gemäß Anspruch 1, in dem die biologische Probe eine Blutprobe oder Serumprobe ist.

3. Verfahren gemäß Anspruch 1 oder 2, in dem das Vorhandensein von Anti-Tenascin C-Antikörpern in der biologischen Probe mit einem Kontrolliert verglichen wird, wobei das Vorhandensein von Anti-Tenascin C-Antikörpern in einer den Kontrollwert übersteigenden Menge auf PAH oder ein Risiko der Entwicklung von PAH hinweist.

4. Verfahren gemäß einem der Ansprüche 1 bis 3, in dem die Menge an Anti-Tenascin C-Antikörpern mit einem Immunassay bestimmt wird.

5. Verfahren gemäß Anspruch 4, in dem der Immunassay eine ELISA-Messung ist.

6. Verfahren gemäß Anspruch 4 oder 5, umfassend das Inkontaktbringen einer biologischen Probe mit einem Protein, umfassend den Alninosäure-Abschnitt 181 bis 290 der humanen Tenascin C-Sequenz, wie in SEQ ID NR: 1 angegeben.

7. Verfahren gemäß einem der Ansprüche 1 bis 6, in dem der Patient ein Mensch ist.

8. Verfahren gemäß einem der Ansprüche 1 bis 7, in dem der Patient an systemischer Sklerose leidet.

9. Verfahren gemäß einem der Ansprüche 1 bis 7, in dem der Patient am Sharp-Syndrom leidet.

10. Verfahren gemäß einem der Ansprüche 1 bis 7, in dem der Patient an systemischen Lupus erythematodes leidet.

11. Verfahren gemäß einem der Ansprüche 1 bis 7, in dem der Patient an idiopathischer PAH leidet.

12. Verfahren gemäß einem der Ansprüche 1 bis 7, in dem PAH mit einer portalen Hypertonie, mit einer angeborenen Herzkrankheit oder mit einer Infektion mit humanem Immunschwächevirus (HIV) assoziiert ist, oder eine postembolische pulmonale Hypertonie ist.

13. Verfahren gemäß einem der Ansprüche 1 bis 7, in dem der Patient eine Person mit einer Prädisposition für eine PAH-Entwicklung ist, wobei besagte Person vorzugsweise eine oder mehrere Mutationen in dem für BMPRII codierenden Gen trägt.

14. *In vitro* Verfahren zur Prognose oder zur Verfolgung von PAH, umfassend die Bestimmung des Vorhandenseins und/oder der Menge an Anti-Tenascin C-Antikörpern in einer von einem Patienten stammenden biologischen Probe zu verschiedenen Zeitpunkten, wobei die Erhöhung der Menge an Anti-Tenascin C-Antikörpern über die Zeit auf eine Verschlimmerung der PAH hinweist.

15. *In vitro* Verfahren zur Evaluation der Wirksamkeit einer Behandlung gegen PAH, umfassend die Bestimmung des Vorhandenseins und/oder der Menge an Anti-Tenascin C-Antikörpern in einer von einem Patienten stammenden biologischen Probe zu verschiedenen Zeitpunkten vor, während und nach der Behandlung, wobei die Verringerung der Menge an Anti-Tenascin C-Antikörpern über die Zeit auf eine Verbesserung der PAH hinweist.
